(19) Europäisches Patentamt
European Patent Office
Office européen des brevets

(11) Publication number: 0 207 599 A1

# (12) EUROPEAN PATENT APPLICATION

(21) Application number: 86303403.9

(22) Date of filing: 06.05.86

(51) Int. Cl.⁴: C 07 C 57/42
C 07 C 69/618, C 07 C 51/00
C 07 C 67/00, A 61 K 31/19
A 61 K 31/215

(30) Priority: 09.05.85 GB 8511759

(43) Date of publication of application: 07.01.87 Bulletin 87/2

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Buckle, Derek Richard
18 Hillfield Road
Redhill Surrey RH1 4AP(GB)

(72) Inventor: Fenwick, Ashley Edward
1 Prospect Villas High Street
Partridge Green West Sussex RH13 8EN(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

## (54) Arachidonic acid analogues.

(57) Compounds of formula (I):

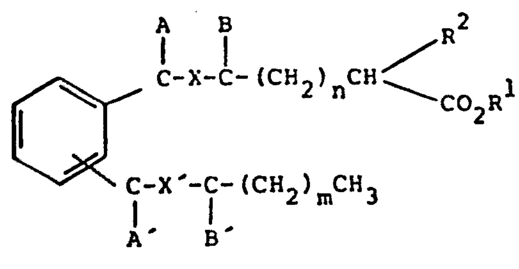

(I)

or, where appropriate, a solt or amide thereof,

wherein $R^1$ is hydrogen or $C_{1-6}$ alkyl;

$R^2$ is hydrogen or $CO_2R^3$ where $R^3$ is hydrogen or $C_{1-6}$ alkyl;

X and X' are independently a double or triple bond;

A and B are hydrogen when X is a double bond or A and B are both absent when X is a triple bond;

A' and B' are hydrogen when X' is a double bond or A' and B' are both absent when X' is a triple bond;

n is 0 or an integer of from 1 to 4; and m is an integer of from 2 to 7; processes for preparing such compounds; pharmaceutical compositions containing such compounds and the use of such compounds and compositions in medicine.

EP 0 207 599 A1

Croydon Printing Company Ltd.

TITLE MODIFIED
see front page

86303403.9

0207599



B1808

NOVEL COMPOUNDS

This invention relates to novel arachidonic acid analogues, to processes for preparing them, to pharmaceutical compositions containing them and the use of such compounds and compositions in medicine.

It is known that certain arachidonic acid metabolites can produce harmful effects in man. For example, some prostaglandins and thromboxanes, produced via cyclo-oxygenation of arachidonic acid, can contribute to inflammation in such diseases as rheumatoid arthritis, and that products produced via lipoxygenation of arachidonic acid, such as the leukotrienes, are implicated in the production of the pathology of asthma and other allergic diseases.

European Published Patent Application No.0109225 discloses certain arachidonic acid analogues which can inhibit arachidonic acid metabolism by one or both of these metabolic pathways.

We have now discovered a new class of arachidonic acid analogues which are inhibitors of phospholipase $A_2$,one of the enzymes involved in the release of arachidonic acid from its phospholipid store. These analogues are thus of value in the prophylaxis and treatment of diseases whose symptoms are controlled by arachidonic acid metabolites.

According to the present invention there is provided a compound of formula (I):

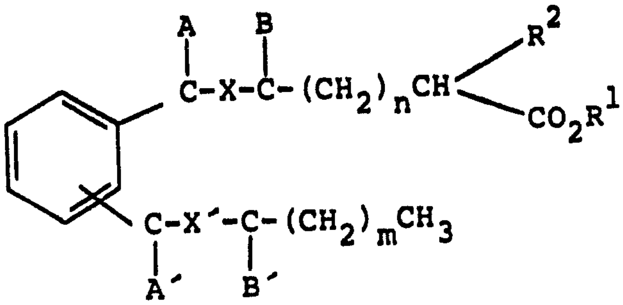

(I)

or, where appropriate, a salt or amide thereof,

wherein $R^1$ is hydrogen or $C_{1-6}$ alkyl;

$R^2$ is hydrogen or $CO_2R^3$ where $R^3$ is hydrogen or $C_{1-6}$ alkyl;

X and X′ are independently a double or triple bond;

A and B are hydrogen when X is a double bond or A and B are both absent when X is a triple bond;

A′ and B′ are hydrogen when X′ is a double bond or A′ and B′ are both absent when X′ is a triple bond;

n is O or an integer of from 1 to 4; and m is an integer of from 2 to 7.

When X is a double bond, the hydrocarbon chain containing X may have the (E) or (Z) absolute configuration but preferably the (Z) configuration.

Similarly when X′ is a double bond, the hydrocarbon chain containing X′ may have the (E) or (Z) absolute configuration but preferably the (Z) configuration.

The side chains are preferably in the ortho or meta positions with respect to each other on the aromatic ring.

Suitably $R^1$ is hydrogen.

Suitably $R^1$ is ethyl.

Suitably $R^2$ is hydrogen, $-CO_2H$,$-CO_2CH_3$, $-CO_2CH_2CH_3$ or $-CH_2Bu$.

Preferably $R^1$ is hydrogen and $R^2$ is hydrogen, $-CO_2H$, $-CO_2CH_3$, $-CO_2CH_2CH_3$ or $-CO_2Bu$.

Preferably n is 2.

Preferably m is 4 or 5.

Salts or amides of the compounds of formula (I) need not be pharmaceutically acceptable, since they can be used as intermediates to prepare other pharmaceutically acceptable compounds of the invention.

Examples of pharmaceutically acceptable salts include alkali metal and alkaline earth metal salts, such as sodium, potassium and magnesium salts; and salts with ammonia, organic bases and amino compounds.

Examples of pharmaceutically acceptable amides are those wherein the carboxyl group is modified to a group $-CONR^3R^{3a}$, wherein $R^3$ and $R^{3a}$ are each hydrogen or $C_{1-6}$ alkyl.

The present invention also encompasses solvates of the compounds of formula (I) or, where appropriate the salts or amides thereof; examples of suitable solvates are hydrates and ethanolates; preferably the solvates are hydrates.

Examples of compounds of formula (I) include:

6-[2-(1-octynyl)phenyl]-E-hex-5-en-1-oic acid;
6-[2-(Z-1-octenyl)phenyl]-E-hex-5-en-1-oic acid;
6-[3-(1-octynyl)phenyl]-E-hex-5-en-1-oic acid;
2-(4-[2-(1-octynyl)phenyl]but-3-ynyl)malonic acid;
2-(4-[2-((Z)-1-octenyl)phenyl-(Z)-but-3-enyl)-malonic acid;
2-(4-[2-(1-octynyl)phenyl]-but-3-ynyl)malonic acid monoterbutyl ester;
6-[2-(1-octynyl)phenyl)hex-5-yn-1-oic ocid; and
6-[12-((Z)-1-octenyl)phenyl]-(Z)-hex-5-en-1-oic acid;

or where appropriate a salt, amide or solvate thereof.

Further examples of compounds of formula (I) include:

diethyl 2-(4-[2-(1-octynyl)phenyl]-but-3-ynyl)-malonate;
diethyl-2-(4-[2-(Z)-1-octenyl)phenyl]-(Z)-but-3-enyl)malonate;
tert butylethyl-2-(4-[2-(1-octynyl)phenyl]-but-3-ynyl)malonate; and
methyl-6-[2-((Z)-1-octenyl)phenyl]-(Z)-hex-5-en-1-oate;
or, where appropriate a salt, amide or solvate thereof

According to a further aspect of the invention there is provided a process for preparing a compound of formula (I) wherein X is a double bond and A and B are hydrogen or a salt, amide or solvate thereof which process comprises reacting a compound of formula (II):

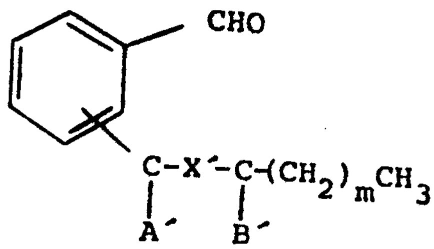

(II)

wherein X′, A′, B′ and m are as defined in relation to formula (I) with a compound of formula (III):

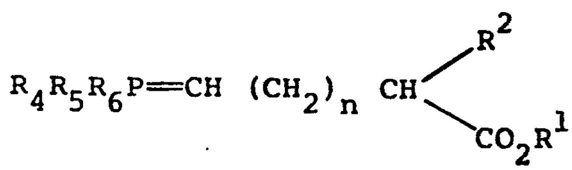

(III)

wherein $R^1$ is hydrogen and $R^2$ and n are as defined in relation to formula (I), and $R^4$, $R^5$ and $R^6$ are each independently $C_{1-6}$ alkyl or aryl such as phenyl; in the presence of a strong base,

and thereafter if desired carrying out one or more of the following steps:

(i) where X′ is a triple bond, reducing the triple bond to a double bond;

(ii) converting one group $R^1$ to another group $R^1$;

(iii) converting one group $R^2$ to another group $R^2$; and

(iv) forming a salt, amide or solvate thereof.

The reaction is suitably carried out in an inert organic solvent such as diethylether, tetrahydrofuran, benzene, toluene, dimethylformamide or dimethylsulphoxide at a temperature of from -20-100°C, suitably at about 0°C. Usually both E and Z isomers are formed, the ratios of each depending on the reaction conditions.

The reaction is suitably carried out under an inert atmosphere of for example nitrogen or argon.

When X′ is a triple bond, hydrogenation thereof has been found to produce as the predominant product, a compound of formula (I), wherein X is a double bond in the cis configuration (i.e.Z) although trans compounds (i.e.E) may be formed as a result of subsequent isomerisation.

The conversion of one group $R^2$ to another such group in optional step (ii) above can be carried out by conventional methods. For example, a compound of formula (I) wherein $R^2$ is hydrogen can be converted to a compound of formula (I) wherein $R^2$ is $CO_2R^3$ by metallation, for example using butyl lithium and subsequent quenching with $C_{1-6}$ alkylchloroformate or carbon dioxide. The reaction is suitably carried out in an inert organic solvent, for example an ethereal solvent such as tetrahydrofuran. Low temperatures of from -78 to 20°C are preferably employed.

A compound of formula (I) wherein $R^2$ is $CO_2H$ can be decarboxylated, for example by heating to give a compound of formula (I) wherein $R^2$ is hydrogen. Temperatures of up to and including the melting point are usually sufficient for this.

Compounds of formula (II) can be prepared by reaction of a compound of formula (IV):

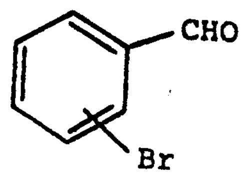

(IV)

with a compound of formula (V):

$$CH_3(CH_2)_mC{\equiv}C\text{-}Z \qquad (V)$$

wherein m is as defined in relation to formula (I)and Z is hydrogen or a metallic cation such as $(ZnCl)^+$; and thereafter if desired reducing the triple bond to a double bond.

When Z represents hydrogen the reaction is suitably carried out under conventional Heck reaction conditions in an organic solvent such as a secondary or a tertiary amine for example triethylamine or tetramethylethylenediamine in the presence of a palladium catalyst. Suitable catalysts include $Pd(OAc)_2[PPh_3]_2$, $Pd\ Cl_2[PPh_3]_2$ and $Pd[PPh_3]_4$, with or without copper (I) iodide as a co-catalyst. Elevated temperatures of from 50-150$^oC$ are suitably employed, preferably under reflux. The reaction is suitably carried out under an inert atmosphere of for example nitrogen or argon.

When Z represents a metallic cation, preferably $(ZnCl)^+$; the reaction is suitably carried out under

conventional cross coupling conditions (see for example E.I. Negishi. Acc. Chem. Res. 1982, 15, (340-348)). Suitably the reaction is carried out in a solvent such as tetrahydrofuran in the presence of an appropriate catalyst, such as $Pd(PPh_3)_4$. Elevated temperatures of from 30-80°C are suitably employed, preferably at 60°C. The reaction is suitably carried out under an inert atmosphere of for example nitrogen or argon.

The optional step of reducing the triple bond to a double bond can be effected using conventional methods, for example by reduction with hydrogen in the presence of a poisoned catalyst such as a Lindlar catalyst or palladium on barium sulphate, under conventional conditions.

Compounds of formula (III) are either known compounds or are suitably generated by reaction of a compound of formula (VI):

$$R_4R_5R_6\overset{+}{P}-CH_2(CH_2)_n\underset{\displaystyle R_2}{\underset{|}{C}}HCO_2R_1-\overset{-}{Y^1} \quad (VI)$$

wherein $R_1$ and $R_2$ are as defined in relation to formula (I), $R_4$, $R_5$ and $R_6$ are as defined in relation to formula (II) and $Y^1$ is halogen such as bromine or iodine; with a strong base.

Examples of suitable bases include alkali metal $C_{1-6}$ alkoxides such as sodium methoxide, sodium ethoxide, potassium butoxide or lithium ethoxide, or sodium dimsyl, organo lithium compounds such as n-butyl lithium.

Compounds of formula (IV),(V) and (VI) are known compounds or can be produced from known compounds by known methods.

Salts, amides and solvates of the compounds of formula (I) may be prepared by methods conventional in the art.

Further according to the present invention there is provided a process for preparing a compound of formula (I) or a salt, amide or solvate thereof which comprises deprotecting a compound of formula (VII):

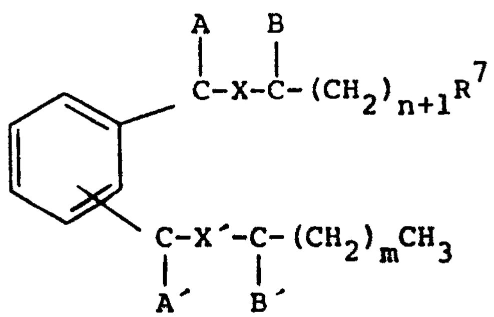

(VII)

wherein X, A, B, X′, A′, B′, m and n are as defined in formula (I) and $R^7$ is a protected acid group; and thereafter if necessary carrying out one or more of the following steps:

(i) where X and/or X′ is a triple bond, reducing it to a double bond;

(ii) converting a group $R^1$ from hydrogen to another group $R^1$;

(iii) converting a group $R^2$ from hydrogen to another group $R^2$; and

(iv) forming a salt, amide or solvate thereof.

Suitable protected acid groups $R^7$ are the methyl trioxabicyclo-octane or oxazoline groups.

The reaction is suitably carried out in an organic solvent such as ether or ethanol in the presence of an acid such as the halogen acids for example hydrochloric acid.

Moderate temperatures of from 0 to 100°C can be employed.

Compounds of formula (VII) can be prepared by reaction of a compound of formula (VIII):

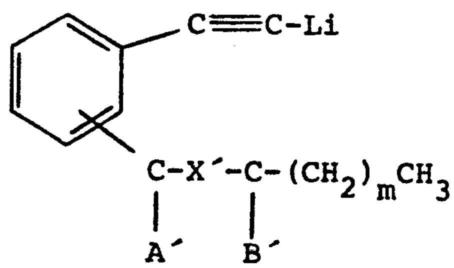

(VIII)

wherein X′, A′, B′ and m are as defined in relation to formula (I) with a compound of formula (IX):

$$Y(CH_2)_{n+1}R^7 \qquad (IX)$$

wherein n is as defined in relation to formula (I), $R^7$ is as defined in relation to formula (VII) and Y is a leaving group such as sulphonyloxy, mesyloxy, tosyloxy or trifluoromethylsulphonyl;

and thereafter if desired reducing the triple bond to a double bond.

The reaction is suitably carried out in an inert organic solvent such as tetrahydrofuran at a temperature of from -40 to 60°C.

Compounds of formula (VIII) can be prepared by reaction of a compound of formula (X):

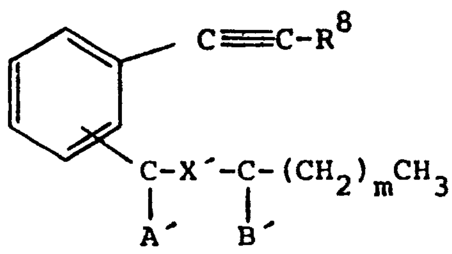

(X)

wherein A', B', X' and m are as defined in relation to formula (I) and $R^8$ is hydrogen, or halogen such as bromine; with an organic lithium compound such as $C_{1-6}$ alkyl lithium, in particular n-butyl lithium.

The reaction is suitably carried out in an organic solvent such as tetrahydrofuran at a temperature of from -78 to 20°C.

Compounds of formula (X) can be prepared by reaction of a compound of formula (XI):

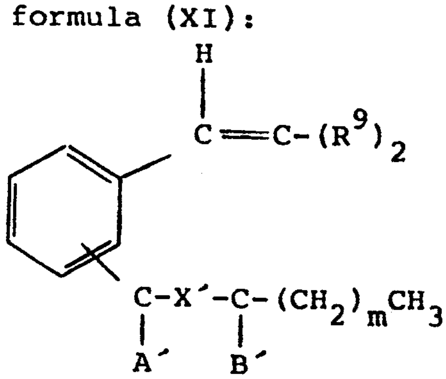

(XI)

wherein A', B', X' and m are as defined in relation to formula (I) and $R^9$ is halogen such as bromine; with a strong base.

Examples of suitable bases include organic alkali metal compounds such as n, sec. or t-butyl lithium which will usually produce a compound of formula (X) wherein $R^8$ is hydrogen, and alkoxides such as potassium amyloxide or sodium methoxide both of which will typically result in a compound of formula (X) wherein $R^8$ is halogen as a predominant product.

The reaction is suitably carried out in an organic solvent such as hexane at a temperature of from -20°C to 100°C.

Compounds of formula (X) wherein $R^8$is halogen can be readily converted to compounds of formula (X) wherein $R^8$ is hydrogen by treatment with an alkyl lithium such as n-butyl lithium under the above described conditions.

Compounds of formula (XI) can be prepared by reaction of a compound of formula (II) as hereinbefore defined with a halogenating agent such as carbontetrabromide in the presence of a trivalent phosphine such as triphenyl phosphine.

The reaction is suitably carried out in an inert organic solvent such as dichloromethane, at a temperature of from -20 to 20°C.

Where the compound of formula (I) required is one in which X′ is a double bond, the compound of formula (II) employed is preferably one in which X′ is a double bond. These may be prepared by catalytic reduction of a compound of formula (II) in which X′is a triple bond by conventional methods, for example as described herein.

Alternatively compounds of formula (VII) as hereinbefore defined can be prepared by reacting a compound of formula (XII):

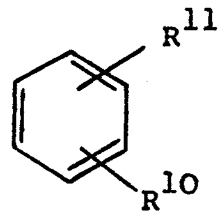

(XII)

wherein $R^{10}$ and $R^{11}$ are each independently a halogen atom such as bromine, with

(a) a compound of formula (V) as hereinbefore defined and

(b) a compound of formula (XIIIa):

```
R10Zn C-X-C-(CH2)n+1R7          (XIIIa)
      |   |
      A   B
```

wherein A, B, X and n are as defined in relation to formula (I), $R^7$ is as defined in relation to formula (VII) and $R^{10}$ is halogen such as chlorine, providing that when Z in the compound of formula (V) is hydrogen the reaction sequence must be (a) followed by (b) and when Z is a metal cation the reaction sequence is (a) followed by (b) or (b) followed by (a); and thereafter if desired converting any triple bonds to double bonds.

Preferably the reactions are carried out in the order (a) followed by (b).

When X is a double bond and A and B are hydrogen the compound of formula (XIIIa) may have either the E or Z configuration, which leads to the stereospecific formation of compounds of formula (I).

When Z represents hydrogen, the reaction step (a) is suitably carried out under conventional Heck reaction conditions as described above in relation to the reaction between the compounds of formula (IV) and the compounds of formula (V) wherein Z represents hydrogen.

When Z represents a metallic cation, the reaction step (a) is suitably carried out under conventional cross-coupling conditions as described above in relation to the reaction between the compounds of formula (IV) and the compounds of formula (V) wherein Z represents a metallic cation.

The reaction step (b) is suitably carried out under conventional cross-coupling conditions, preferably in the presence of a palladium catalyst.

Compounds of formula (X) as defined above wherein $R^8$ is hydrogen can also be prepared from compounds of formula (XII) as defined above by reaction in any order with (c) a compound of formula (XIV):

$$H-C\equiv C-R^{12} \qquad (XIV)$$

wherein $R^{12}$ is a protecting group such as a silyl protecting group, for example trimethylsilyl, and (d) a compound of formula (XIIIb):

$$R^{10}ZnC-X'-C-(CH_2)_mCH_3 \qquad \text{(XIIIb)}$$

A′ B′

wherein A′, B′ X′ and m are as defined in relation to formula (I) and $R^{10}$ is as defined in relation to formula (XIIIa); and thereafter if desired removing any protecting group $R^{12}$ for example with a base and reducing any triple bonds to a double bond.

The reaction of step (c) is suitably carried out under conventional Heck reaction conditions as described above.

The reaction of step (d) is suitably carried out under conditions similar to that described in step (b) above.

Alternatively compounds of formula (X) where $R^8$ is hydrogen and X′ is a triple bond can be prepared by reacting a compound of formula (XII) as hereinbefore defined in any order with (e) a compound of formula (V) as hereinbefore defined and (f) a compound of formula (XV):

$$H-C{\equiv}C-R^{12a} \qquad \text{(XV)}$$

wherein $R^{12a}$ is hydrogen or a group $R^{12}$ as defined above in relation to formula (XIV) and thereafter if desired removing any protecting groups $R^{12}$ for example by reaction with a base.

Steps (e) and (f) are suitably carried out under conventional Heck reaction conditions as described above. When $R_{12}$ is hydrogen, reaction (f) should be carried out under cross-coupling conditions.

In another aspect, compounds of formula (VII) as hereinbefore defined can be prepared by reaction of a compound of formula (XVI):

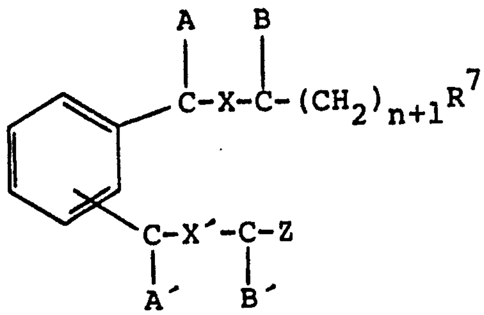

(XVI)

wherein A, B, X, A′, B′, X′ and n are as defined in relation to formula (I) $R^7$ is as defined in relation to formula (VII) and Z is a metal ion or salt thereof such as ZnCl;

with a compound of formula (XVII):

$$R^{13}(CH_2)_mCH_3 \qquad (XVII)$$

wherein m is as defined in relation to formula (I) and $R^{13}$ is a leaving group. Suitable leaving groups $R^{13}$ include iodo, bromo, tosyl, mesyl and trifluorosulphonyl.

The reaction is suitably carried out in an organic solvent such as tetrahydrofuran at a temperature of from -30 to 60°C.

Compounds of formula (XVII) are known compounds or can be prepared from known compounds by known methods.

Compounds of formula (XVI) can be prepared by metallation of a compound of formula (XVIII):

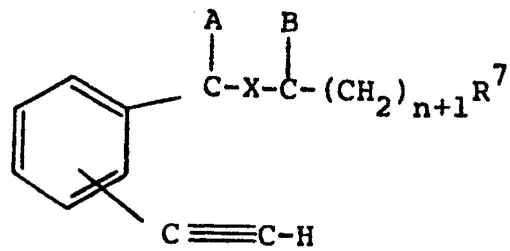

(XVIII)

wherein A, B, X, $R^7$ and n are as defined above, optionally with concomitant reduction of the alkyne.

A suitable reducing agent is diisobutylaluminium hydride (dibal) which results in the stereoselective reduction of the triple bond to a double bond.

Suitable reagents for effecting the metalation include lithiation with for example n-butyl lithium followed optionally by transmetalation with for example $ZnCl_2$. The reaction is suitably carried out in an inert organic solvent such as tetrahydrofuran at a temperature of from -78 to 20°C.

When the reduction is carried out in a non-polar solvent such as heptane, the double bond formed has predominently the cis configuration. In contrast, when the reaction is carried out in a polar solvent such as diethylether the double bond formed is predominently in the trans configuration.

Compounds of formula (XVIII) can be prepared from compounds of formula (XII) as hereinbefore defined by methods analogous to those described above. For example, the compound of formula (XII) can be reacted in any order with a compound of formula (XIIIa) and a compound of formula (XV) as hereinbefore defined, and thereafter any protecting groups removed.

Further according to the present invention there is provided a process for preparing a compound of formula (I) or a salt, amide or solvate thereof which process comprises reacting a compound of formula (XIX):

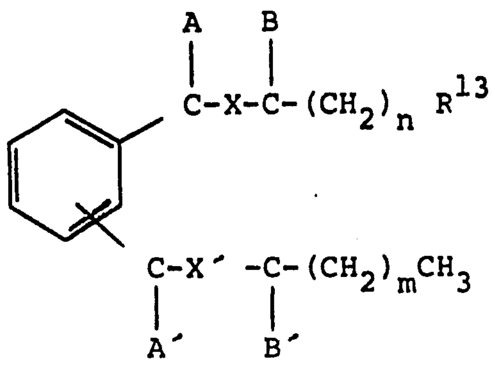

(XIX)

wherein A,B,X,A',B',X', n and m are as defined in relation to formula (I) and $R^{13}$ is a leaving group with a source of malonate ions of formula (XX):

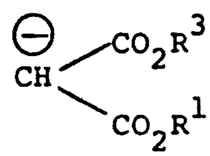

(XX)

and thereafter if desired carrying out one or more of the following steps:

(i) reducing any triple bonds to double bonds;

(ii) converting a group $R^2$ from $CO_2R^3$ to another group $R^2$; and

(iii) forming a salt, amide or solvate thereof.

Suitable leaving groups $R^{13}$ include those defined in relation to formula (XVII), and sulphonyloxy groups for example of formula $OSO_2R^{14}$ where $R^{14}$ is $C_{1-6}$ alkyl, such as methyl, or a tolyl group such as p-tolyl.

A suitable source of malonate ions of formula (XX) is provided by the appropriate malonate in the presence of sodium hydride or other appropriately strong bases.

The reaction is suitably carried out in an inert organic solvent such as tetrahydrofuran at a temperature of from 0 to 80°C.

Compounds of formula (XIX) can be prepared by conversion of the hydroxy group of a compound of formula (XXI):

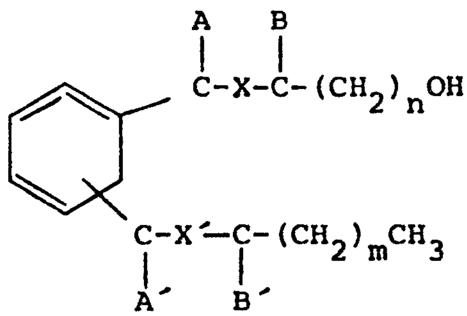

(XXI)

wherein A,B,X, A′,B′,X′, m and n are as defined in relation to formula (I); to a leaving group $R^{13}$ as defined above.

The reaction is carried out by conventional methods, for example using a reagent of formula $R^{15}$ $R^{13}$ wherein $R^{13}$ is as defined above and $R^{15}$ is halogen or a suitable halogenating agent. A preferred example of such a reagent is $ClSO_2R^{14}$ wherein $R^{14}$ is as defined above.

Compounds of formula (XXI) can be prepared from compounds of formula (VIII) as hereinbefore defined by reaction with either (g) a $C_{1-4}$ alkylene oxide such as ethylene oxide or (h) a protected $C_{1-4}$ alkyl halide such as α, trimethylsilyloxy $C_{1-4}$ alkyl iodide, the silyl group being removable using an acid. Any triple bonds may then optionally be reduced to double bonds.

These reactions are carried out under conventional conditions.

For example, reaction (g) above can be carried out in an inert organic solvent such as tetrahydrofuran at a temperature of from 0 to 60°C.

Alternatively the reaction (h) above can be carried out in an inert organic solvent such as tetrahydrofuran at a temperature of from 0 to 60 °C; optionally followed by treatment with an acid such as hydrochloric acid.

Further according to the present invention there is provided a process for preparing a compound of formula (I) or a salt, amide or solvate thereof which comprises oxidation of a compound of formula (XXII):

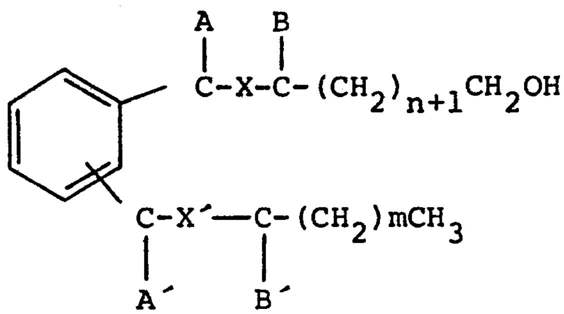

(XXII)

wherein A,B,X,A',B',X' n and m are as defined in relation to formula (I) and thereafter if desired carrying out one or more of the following steps:

(i) where X and/or X' is a triple bond, reducing the triple bond to a double bond;

(ii) converting one group $R^1$ to another such group;

(iii) converting one group $R^2$ to another such group; and

(iv) forming a salt, amide, or solvate thereof.

The oxidation is suitably carried out using a conventional oxidising agent such as $KMnO_4$ or $CrO_3$. The reaction is suitably carried out in an inert solvent such as acetic acid, water or pyridine at a temperature of from 0 to 20 °C.

Compounds of formula (XXII) can be prepared by methods analogous to those described above for the preparation of compounds of formula (XXI).

Yet further according to the present invention there is provided a process for preparing a compound of formula (I) or a salt amide, or solvate thereof which process comprises reaction of a compound of formula (XXIII):

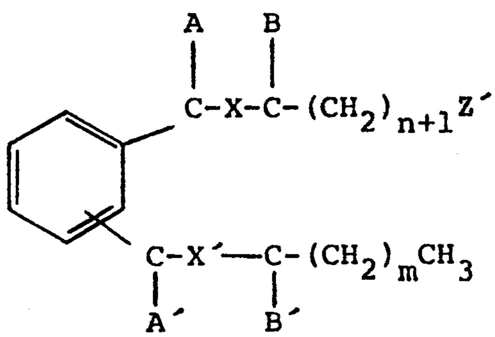

(XXIII)

where A,B,X,A′,B′,X′, m and n are as defined in relation to formula (I) and Z' is a metallic cation or metal salt ion; with carbon dioxide, or a suitable chloroformate and thereafter if required carrying out one or more of the following steps:

(i) converting a group $R^2$ from hydrogen to another group $R^2$;

(ii) reducing any triple bonds to double bonds; and

(iii) forming a salt, amide or solvate thereof.

Suitably Z′ is the $(MgCl)^+$ ion.

The reaction is suitably carried out in an organic solvent such as ether or tetrahydrofuran of from -78 to 50°C.

Compounds of formula (XXIII) can be prepared by metal halogen exchange reaction of a compound of formula (XXIV):

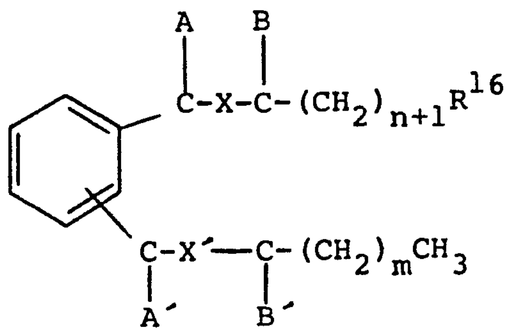

(XXIV)

wherein A,B,X,A′,B′,X′, n and m are as defined in relation to formula (I) and $R^{16}$ is halogen such as bromide or iodide, or by Grignard formation.

Conventional metal-halogen exchange reagents such as nBuLi or magnesium can be employed using conventional conditions.

Compounds of formula (XXIV) can be prepared from compounds of formula (XXII) by conventional halogenation reactions as for example $CBr_4/Ph_3P$.

The compounds of formula (I) are active therapeutically, and, accordingly, this invention also provides a compound of formula (I), or a pharmaceutically acceptable salt, amide or solvate thereof, for use as an active therapeutic substance. In particular the present invention provides a compound of formula (I), or a pharmaceutically acceptable salt, amide or solvate thereof, for use in the treatment of allergic and/or inflammatory diseases.

The compounds of the invention may be administered alone or as a pharmaceutical composition also comprising a pharmaceutically acceptable carrier.

Accordingly, the present invention also provides a pharmaceutical composition comprising a compound of formula (I), or a pharmaceutically acceptable salt, amide, or solvate thereof, and a pharmaceutically acceptable carrier therefor.

A compound of formula (I), or a salt, amide or solvate thereof, which is active when given by the oral route, may be compounded in the form of syrups, tablets, capsules, pills and the like. Preferably, the composition is in unit dosage form, or in a form in which the patient can administer to himself a single dosage. When the composition is in the form of a tablet, powder or lozenge, any pharmaceutical carrier suitable for formulating solid compositions may be used. Examples of such carriers are magnesium stearate, starch, lactose, glucose,sucrose, rice flour and chalk. The composition may also be in the form of an ingestible capsule (e.g. of gelatin) containing the compound; or in the form of a syrup, a liquid solution or a suspension. Suitable liquid pharmaceutical carriers include ethyl alcohol, glycerine, saline and water which may be compounded with flavouring or colouring agents to form syrups.

The compounds of this invention may also be administered by a non-oral route. In accordance with routine pharmaceutical procedure, the compositions may be formulated, for example for rectal administration as a suppository or for presentation in an injectable form in an aqueous or non-aqueous solution, suspension or emulsion in a pharmaceutically acceptable liquid, e.g.

sterile pyrogen-free water or a parenterally acceptable oil or a mixture of liquids, which may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi- dose forms such as a bottle from which the appropriate dose may be withdrawn or a solid form or concentrate which can be used to prepare an injectable formulation.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate, small amounts ofother anti-asthmatics and bronchodilators, for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

A compound of general formula (I) or salt, amide or solvate thereof,may also be presented as an ointment, cream, lotion, gel, aerosol, or skin paint for topical application.

It is preferred that the compounds of this invention are administered by inhalation.

By way of example, in any of the preceding formulations a suitable dosage unit might contain 0.01 to 500mgs of active ingredient, more suitably 1 to 500mgs via the oral route, 0.01 to 10mgs via inhalation. The effective dose of compound depends on the particular compound employed, the condition of the patient and on the frequency and route of administration, but in general is in the range of from 0.001 mg/kg/day to 100 mg/kg/day inclusive of the patient's body weight.

Within the above mentioned dosage range, no adverse toxicological effects are indicated with the compounds of the invention.

As in common practice, the compositions will usually be accompanied by written or printed directions for use in the medical treatment concerned, in this case as an anti-allergic agent for the prophylaxis and treatment of, for example, asthma, hay fever, rhinitis or allergic eczaema, or as an anti-inflammatory agent for the treatment of, for example rheumatoid arthritis.

The invention also provides a method for treating allergic and/or inflammatory diseases in human and non-human animals, which comprises administering to the sufferer an effective non toxic amount of a compound of formula (I) or a pharmaceutically acceptable salt, amide or solvate thereof.

In a further aspect the present invention provides the use of a compound of formula (I), or a pharmaceutically acceptable salt, amide or solvate thereof, for the manufacture of a medicament for the treatment of allergic and/or inflammatory diseases.

The following Examples illustrate the preparation of compounds of this invention.

EXAMPLE 1

2-(1-Octynyl)benzaldehyde

A mixture of the 2-Bromobenzaldehyde (7.4g, 40mmole), triphenylphosphine (0.57g), palladium (II) acetate (0.16g) and oct-1-yne (8g, 1.8eq) in triethylamine (150ml) was degassed and heated at 110°C under nitrogen overnight. After cooling the precipitate of triethylamine hydrobromide (7.28g, 100%) was removed by filtration and was washed with diethyl ether. The filtrate was concentrated under reduced pressure to give a red oil (19g) which was chromatographed on silica (DCM: Hexane 1:3) to give the title compound (5.28g, 62%) as a yellow oil. $\nu_{max}$ (film) 2220 (w), 1700 (s), 1595 (m), 1485 (m), 1190 (m), 765 (m); $\delta$($CDCl_3$) 0.83 (3H, distorted t, terminal $CH_3$), 1.20 (8H, br. alkylene chain), 2.31 (2H, t, J = 6Hz, $-CH_2$ C≡C-), 7.15-7.9 (4H, m, aromatic), 10.43 (1H, s, -CHO) characterised as the 2,4-dinitrophenylhydrazone, m.p. 143°C.

Found; C, 63.83; H, 5.56; N, 14.15 $C_{21}H_{22}N_4O_4$
requires; C, 63.95; H, 5.62; N, 14.20%

EXAMPLE 2

6-[2-(1-Octynyl)phenyl]-E-hex-5-en-1-oic acid

t-Amyl alcohol (4.22g, 48mmole) in THF (25ml) was added to sodium hydride (1.15g, 48mmole) at room temperature. When the ensuing effervescence had ceased, the solvent was removed under reduced pressure and was replaced with benzene (25ml). The resulting solution was added to a suspension of 4-(carboxy butyl) triphenyl phosphonium bromide (10.56g, 24mmole) and DMSO (0.72ml) in benzene (48ml) at 60$^{o}$C. After 15 minutes a solution of 2-(1-octynyl) benzaldehyde (2.58g, 12mmole, from Example 1) was added to the orange suspension. The colour was immediately discharged and the suspension then rapidly darkened. After 15 minutes the mixture was added to ice/HCl (20ml) the dark colour immediately going, and the resulting mixture was extracted with diethyl ether (2 x 100ml). The ether layer was washed with brine, dried ($MgSO_4$) and evaporated to give an oil which was chromatographed on 10% argentated silica (DCM:$Et_2O$) to give the title compound (2.19g, 64%) as an oil. $\nu_{max}$ (film) 2940 (s), 2220 (w), 1710 (s), 970 (m), 750 (m) $cm^{-1}$; $\delta$($CDCl_3$) 0.90 (3H, t, $\underline{J}$ = 5.4Hz, terminal $CH_3$), 1.2-1.7 (8H, m, alkylene chain), 1.84 (2H, quintet, $\underline{J}$ = 7.42Hz, $\underline{CH_2}CH_2CO_2H$), 2.31 (2H, q, $\underline{J}$ = 7.56Hz, $\underline{CH_2}CH_2CH_2CO_2H$), 2.42 (2H, t, $\underline{J}$ = 7.42 Hz $\underline{CH_2}CO_2H$), 2.46 (2H, t, $\underline{J}$ = 7.13Hz, C≡C-$CH_2$), 6.22 (1H, dt, $\underline{J}$t = 7.13Hz, $\underline{J}$d = 11.2Hz, A-CH=$\underline{CH}$-), 6.9 (1H, d, $\underline{J}$ = 16.2Hz, Ar-$\underline{CH}$=CH-), 7.05-7.5 (4H, m, aromatic), 9.1-10.4 (1H, br, COOH); $M^{+}$ 298.1926, $C_{20}H_{26}O_2$ requires 298.1933.

Found; C, 80.76; H, 8.75; $C_{20}H_{26}O_2$
requires; C, 80.50; H, 8.78%.

EXAMPLE 3

6-[2-(Z-1-Octenyl)phenyl]-E-hex-5-en-1-oic acid

A mixture of 6-[2-(1-octynyl)phenyl]-E-hex-5-en-1-oic acid (1.08g, 3.6mmole, from Example 2), pyridine (25ml) and 5% palladium on barium sulphate (0.06g) was hydrogenated at atmospheric pressure. The hydrogen uptake stopped when 87ml of hydrogen had been absorbed Filtration through celite and evaporation of the solvent at reduced pressure gave an oil which was chromatographed on 10% argentated silica to give the title compound (0.53g, 49%), as an oil. $\nu_{max}$ (film) 2920 (s), 1710 (s), 1710 (s), 965 (m), 750 (m) $cm^{-1}$, $\delta(CDCl_3)$ 0.85 (3H, t, J = 7.13Hz, terminal $CH_3$), 1.1-1.7 (8H, m, alkylene chain), 1.83 (2H, quintet, J = 7.14Hz $CH_2CH_2CO_2H$), 2.09 (2H, q, J = 7.5Hz, $-CH = CH-CH_2-R$), 2.27 (2H, t, J = 6.87, $-CH = CH-CH_2-(CH_2)_2CO_2H$), 2.41 (2H, t, J = 7.56Hz, $CH_2CO_2H$), 5.73 (1H, dt, Jd = 11.45Hz, Jt = 7.34Hz, Ar-CH = CH=CH-R), 6.08 (1H, dt, Jd = 15.66Hz, Jt = 6.87Hz, CH = $CH(CH_2)_3CO_2H$), 6.46 (1H, d, J = 11.45Hz, Ar-CH = CH-R), 6.56 (1H, d, J = 15.66Hz, Ar-CH = CH R), 7.05-7.5 (4H, m, aromatic). $M^{+}$ 300.2080, $C_{20}H_{28}O_2$ requires 300.2089.

Found; C, 79.45; H, 9.13; $C_{20}H_{28}O_2$ requires; 79.96; H, 9.39%.

EXAMPLE 4

3-(1-Octynyl)benaldehyde

This reaction was carried out in an identical manner to Example 1 except 3-bromobenzaldehyde (7.4g, 40mmole) was used. The product (5.2g, 61%) was obtained as a yellow oil. $\nu_{max}$ (film) 2950 (s), 2240 (m), 1710 (s), 1600 (m), 1575 (m), 1280 (m), 1160 (m), 800 (m), 690 (m) $cm^{-1}$, $\delta$($CDCl_3$) 0.90(3H, distorted t, terminal $CH_3$), 1.36 (8H, br, alkylene chain), 2.36 ( 2H, t, J = 5Hz, $CH_2$-C≡C), 7.3-7.85 (4H, m, aromatic), 9.8 (1H, s, CHO), characterised as the 2,4-dinitrophenylhydrazone.

EXAMPLE 5

6-[3-(1-Octynyl)phenyl]-E-hex-5-en-1-oic acid

The reaction was carried out in an identical manner to Example 2 except 3-(Oct-1-ynyl)benzaldehyde (3g, 14mmole from Example 4) was used. The product (2.49g, 60%) was obtained as a low melting solid, m.p. 34-40$^{o}$C. $\nu_{max}$ (film) 2930 (s), 2220 (w), 1715 (s), 1600 (m), 960 (m) 685 (m) $cm^{-1}$; $\delta$($CDCl_3$) 0.90 (3H, t, J = 6.48Hz, terminal $CH_3$), 1.25-1.67 (8H, m, alkyledene chain), 1.81 (2H, quintet, J = 6.48Hz, $C\underline{H}_2CH_2CO_2H$), 2.26 (2H, q, J = 6.91Hz, $-CH = CH-C\underline{H}_2-$), 2.39 (4H, t, J = 7.15 Hz $C{\equiv}C-C\underline{H}_2-$, $-C\underline{H}_2-CO_2H$), 6.16 (1H, dt, Jd = 15.88Hz Jt = 6.91Hz, Ar-CH=C$\underline{H}$-), 6.35 (1H, d, J = 15.88Hz, ArC$\underline{H}$=CH), 7.1-7.4 (4H, m, aromatic), 10.2-11.5 (1H, br, COOH); $M^{+}$ 298.1920, $C_{20}H_{26}O_2$ requires 298.1933;

Found; C, 80.59; H, 8.58, $C_{20}H_{26}O_2$ requires; C, 80.50; H, 8.78%.

EXAMPLE 6

2-[2-(1-Octynyl)phenyl]-1,1-dibromo ethene

To a solution of 2-(1-octynyl)benzaldehyde (6.0g, 28mmole from Example 1) in DCM (60ml), containing triphenylphosphine (14.7g, eq) at $5^{O}C$, was added carbontetrabromide (10.7g, 1.15eq) at a rate such that the temperature remained less than $5^{O}C$. After 1hr at $5^{O}C$, the mixture was warmed to room temperature and stirred overnight. The solid present was filtered off and the solvent removed under reduced pressure. Extraction of the residue with n-hexane followed by evaporation yielded an oil, which was chromatographed on silica (hexane) to yield the title compound (7.75g, 77%). $\nu_{max}$ (film) 2940 (s), 2225 (w), 1600 (w), 1470 (m), 885 (m), 860 (s), 785 (m), 750 (s) $cm^{-1}$; $\delta(CDCl_3)$ 0.90(3H, distorted triplet, terminal $CH_3$), 1.15-1.80 (8H, m, alkylene chain), 2.40(2H, t, J = $7H_3$, $C{\equiv}C{-}CH_2$), 7.15-7.75 (4H, m, aromatic), 7.80(1H, s, -CH = $CBr_2$); $M^{+}$ 367.9793, $C_{16}H_{18}Br_2$ requires 367.9776.

Found; C, 51.53; H, 4.85; Br, 43.19; $C_{16}H_{18}Br_2$ requires; C, 51.92; H, 4.90; Br, 43.18%.

EXAMPLE 7

[2-(1-Octynyl)phenyl]ethyne

To a solution of 2-[2-(1-octynyl)phenyl]-1,1-dibromo ethene (0.33g, 0.9mmole from Example 6) in THF (5ml) at -78°C was added a solution of an alkyl lithium in hexane (2eq). After 1hr the dark red solution was added to ice/HCl (10ml) and was extracted into diethyl ether (2 x 50ml). The ethereal solution was dried ($MgSO_4$) and evaporated to give an oil which was chromatographed on silica (hexane) to give the title compound (0.033g, 17.5%) as an oil. $\nu_{max}$ (film) 3290 (m), 2920 (s), 2220 (w), 1595 (w), 1480 (m), 1440 (m), 1340 (w), 755 (s) $cm^{-1}$; $\delta(CDCl_3)$ 0.90 (3H, distorted triplet, terminal $CH_3$), 1.15-1.80(8H, m, alkylene chain), 2.43 (2H, t, J = 7Hz $C{\equiv}C-CH_2$), 3.25 (1H, s, $C{\equiv}C-H$), 7.15-7.55 (4H, m, aromatic); $M^{+}$ 210.1414, $C_{16}H_{18}$ requires 210.1408.

Found; C, 90.94; H, 8.75; $C_{16}H_{18}$ requires; C, 91.37; H, 8.63%.

EXAMPLE 8

2-[2-(1-Octynyl)phenyl]-1-bromo ethyne

To a solution of 2-[2-(1-octynyl)phenyl]-1,1-dibromo ethene (2.96g, 8mmole from Example 6), in n-hexane (15ml) at $0^{o}C$ was added potassium tertamylate (2.05g, 16mmole) suspended in n-hexane (2ml). After 30 minutes the temperature was raised to room temperature and the mixture was stirred overnight. The mixture was added to ice/HCl and was extracted into diethyl ether. The ethereal layer was then washed with brine, dried ($MgSO_4$) and evaporated to give an oil which was chromatographed on silica (n-hexane) to give the title compound (1.58g, 68%) as an oil. $\nu_{max}$ (film) 2920 (s), 2220 (w), 2195 (w), 1595 (w), 1480 (m), 1445 (m), 755 (s) $cm^{-1}$; $\delta$ ($CDCl_3$) 0.90 (3H, distorted triplet, terminal $CH_3$), 1.15-1.90(8H, m, alkylene chain), 2.43 (2H, t, $\underline{J}$ = 7Hz $C{\equiv}C{-}CH_2$), 7.10-7.60(4H, m, aromatic); $M^{+}$ 288.0517. $C_{16}H_{17}Br$ requires 288.0514.

Found: C, 66.67; H, 5.96; Br, 26.89; $C_{16}H_{17}Br$ requires; C, 66.45; H, 5.93; Br, 27.63%.

EXAMPLE 9

[2-(1-Octynylphenyl]ethyne

To 2-[2-(1-octynyl)phenyl]-1-bromoethyne (0.289g, 1mmole from Example 8) in THF (10ml) at -78°C under nitrogen, was added n-butyl lithium (1.1eq). After 2hr at -78°C, the dark solution was added to dilute ice/HCl and was extracted with diethyl ether. The ethereal layer was dried ($MgSO_4$) and evaporated to give an oil (0.21g), which was chromatographed on silica (hexane) to give the title compound (0.18g, 86%) as a colourless oil, identical with that prepared in Example 7.

EXAMPLE 10

4-[2-(1-Octynyl)phenyl]-4-butyn-1-ol

To 2-[2-(1-octynylphenyl]-1-bromo ethyne (2.3g, 11mmole, from Example 8) in THF (25ml) at -78°C was added n-butyl lithium (1eq). After stirring under nitrogen at -78°C for 2hr ethylene oxide (1.3ml, 5eq) was added. After 1hr the temperature was slowly raised to room temperature when more ethylene oxide (1.3ml 5eq) was added. Stirring was continued for 48hr, when the still red solution was added to dilute HCl and was extracted into diethyl ether. After drying the ethereal layer ($MgSO_4$), evaporation of the solvent gave an oil which was chromatographed on silica (DCM: hexane) to give the title compound (1.83g, 65.5%) as an oil. $\nu_{max}$ (film) 3375 (m, br), 2930 (s), 2230 (w), 1590 (w), 1480 (s), 1045 (s), 755 (s) $cm^{-1}$; $\delta(CDCl_3)$ 0.91 (3H, t, J = 6.59Hz terminal $CH_3$), 1.26-1.53 (6H, m, alkylene chain), 1.63 (2H, quintet, J = 6.96Hz, $C{\equiv}C-CH_2\underline{CH_2}R$), 2.40 (1H, t, J = 5.8Hz-O H), 2.45 (2H, t, J = 7.1Hz, $C{\equiv}C-\underline{CH_2}CH_2R$), 2.73 (2H, t, J = 6Hz $C{\equiv}C-\underline{CH_2}CH_2OH$), 3.81 (2H, q, J = 5.86Hz, $CH_2OH$), 7.20 (2H, m, aromatic), 7.39 (2H, m, aromatic); $M^{+}$. 254.1668 $C_{18}H_{22}O$ requires 254.1671.

Found; C, 84.55; H, 9.02; $C_{18}H_{22}O$ requires; C, 84.99; H, 8.72%.

EXAMPLE 11

4-[2-(1-Octynyl)phenyl]-4-butyn-1-ol p-toluene sulphonate

To a solution of 4-[2-(1-octynyl)phenyl]-4-butyn-1-ol (1.5g, 5.9mmole from Example 10) in dry pyridine (20ml) at 0$^{o}$C was added tosyl chloride (2.25g, 2eq). After 2 days at 0$^{o}$C the precipitate of pyridine hydrochloride was filtered off, and the solvent removed under reduced pressure to give an oil. The oil was dissolved in diethyl ether, and the ethereal solution was washed with dilute HCl, dried ($MgSO_4$) and evaporated. The resulting oil was chromatographed on silica (DCM:Hexane) gave the desired tosylate (1.26g, 52.5%) as an oil. $\nu_{max}$ (film) 2930 (s), 2230 (w), 1600 (m), 1370 (s), 1180 (s), 980 (s), 760 (s), 665 (m) $cm^{-1}$; $\delta$($CDCl_3$) 0.90(3H, distorted triplet, terminal $CH_3$) 1.1 - 1.70(8H, m, alkylene chain), 2.40(5H, s, aromatic Me and C≡C-$\underline{CH_2}$-R), 2.81 (2H, t, J=7Hz, C≡C-$\underline{CH_2}CH_2$O-), 4.20(2H, t, J=7.0Hz, C≡C-$CH_2\underline{CH_2}$O-), 7.10- 7.50 (6H, m, aromatics) 7.86 (2H, d, J=8Hz, part of toluene ABq).

Found; C, 73.28; H, 6.83; $C_{25}H_{28}O_3S$
requires: C, 73.50; H, 6.91%.

EXAMPLE 12

Diethyl 2-(4-[2-(1-octynyl)phenyl ]-but-3-ynyl) malonate

To sodium hydride (0.072g, 1.2eq) in THF (5ml) at 0°C was slowly added diethylmalonate (0.48g, 1.2eq). After five minutes reaction had ceased, and after a further five minutes 4-[2-(1-octynyl)phenyl]-4-butyn-1-ol p-toluenesulphonate (1.02g, 2.5mmole from Example 12) in THF (2ml) was added. The mixture was heated to reflux for 36hr when it was added to dilute HCl and was extracted into diethyl ether. The ethereal layer was dried ($MgSO_4$), and was evaporated to give an oil which was chromatographed on silica (DCM) to give the title compound (0.62g, 62.5%) as an oil. $\nu_{max}$ (film) 2925 (s), 2210 (w), 1750 (s), 1735 (s), 1245 (m), 1150 (m), 755 (m) $cm^{-1}$; $\delta(CDCl_3)$ 0.90 (3H, t, J=6.6Hz, terminal $CH_3$), 1.23 - 1.50(12H, m, ester Me and alkylene chain), 1.62 (2H, quintet, J=7.13Hz, $C{\equiv}C{-}CH_2\underline{CH_2}R$), 2.24 (2H, q, J=7.13Hz, $\underline{CH_2}CH(CO_2Et)_2$), 2.47 (2H, t, J=7.13Hz, $C{\equiv}C{-}\underline{CH_2}R$), 2.57 (2H, t, J=6.91Hz, $C{\equiv}C{-}\underline{CH_2}CH_2CH(CO_2Et)_2$), 3.71 (1H, t, J=7.42Hz, $\underline{CH}(CO_2Et)_2$), 4.20 (2H, t, J=7.13Hz, ester $CH_2$), 4.22 (2H, t, J=7.13Hz, ester $CH_2$), 7.18 (2H, m, aromatic), 7.33 (2H, m, aromatic).

Found; C, 75,51; H, 8.15; $C_{25}H_{32}O_4$
requires; C, 75.73; H, 8.14%.

Also formed in the reaction was 1- 2(1-octynyl)phenyl -but-3-yn- 1-ene (0.16g, 15.7%), $\delta(CDCl_3)$, 0.90 (3H, t, J=6.6Hz, terminal $CH_3$), 1.28 - 1.54 (6H, m, alkylene chain), 1.63 (2H, quintet, J=6.96Hz, $C{\equiv}C{-}CH_2\underline{CH_2}R$), 2.46 (2H, t, J=7.1Hz, $C{\equiv}C{-}CH_2$), 5.55 (1H, dd, Ja=10.98, Jc=2.29, 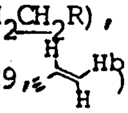), 5.74 (1H, dd, Ja=17.57, Jb=2.29, 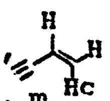 ), 6.06 (1H, dd, Jb=10.98, Jc=17.57, 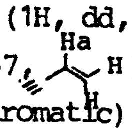), 7.16 - 7.21 (2H, m, aromatic), 7.36 - 7.43 (2H, m, aromatic).

Found; C, 91.23; H, 8.60; $C_{18}H_{20}$
requires; C, 91.46; H, 8.60%.

EXAMPLE 13

2-(4-[2-(1-octynyl)phenyl]-but-3-ynyl)malonic acid.

To a solution of diethyl 4-[2-(1-octynyl)phenyl]-but-3-ynyl malonate (0.43g, 1.1mmole from Example 12) in THF/$H_2O$ (20ml, 50:50) was added lithium hydroxide monohydrate (1g, 10eq). The mixture was stirred under nitrogen at reflux overnight. After cooling and acidifying (dilute HCl) the mixture was extracted into diethyl ether. The ethereal solution was washed with water, dried ($MgSO_4$) and evaported to give a solid (0.33g) m.p. 52-53.5°C. $\nu_{max}$ ($CHCl_3$) 3050 (m, br), 2940 (s), 2650 (m, br), 2225 (w), 1725 (s), 1485 (m), 1445 (m), 1415 (m), 1245 (ms, 1235 (m); $\delta$($CDCl_3$) 0.88 (3H, t, $\underline{J}$ = 6.48Hz, terminal $CH_3$), 1.25-1.50(6H, m, alkylene chain), 1.61 (2H, quintet, $\underline{J}$ = 7.13Hz, C≡C-$CH_2\underline{CH_2}$R), 2.26 (2H, q, $\underline{J}$ = 7.13Hz, $\underline{CH_2}CH(CO_2H)_2$), 2.46 (2H, t, $\underline{J}$ = 7.13Hz, C≡C-$\underline{CH_2}$-R), 2.63 (2H, t, $\underline{J}$ = 6.59Hz, $\underline{CH_2}CH_2CH(CO_2H)_2$), 3.87 (2H, t, $\underline{J}$ = 7.45Hz, $\underline{CH}(CO_2H)_2$), 7.18 (2H, m, aromatic), 7.32 (2H, m, aromatic), 10.76 (2H, br, $CO_2H$).

0207599

## Example 14

## Diethyl-2-(4-[2-(Z)-1-octenyl)phenyl]-(Z)-but-3-enyl) malonate

Diethyl-2-(4-[2-(1-octynyl)phenyl]-but-3-ynyl)malonate (0.99g, 2.5mmole) from Example 12 was hydrogenated in the presence of 5% palladium on barium sulphate (0.1g) according to the method of Example 3. Chromatography on silica (hexane: DCM, 2:1) yielded the title compound (0.84g, 83.5%) as an oil. ν max(film)2930(s), 2890(m), 1750(s), 1635(w), 1590(w) and 770cm$^{-1}$; δ ($CDCl_3$) 0.86(3H, t, J=7.0Hz, terminal $CH_3$), 1.1-1.4(14H, m, alkylene chain + ester $CH_3$), 2.0(2H, q, J=7.7Hz, $CH_2$ $CH(CO_2Et)_2$), 2.18(4H, m, Ar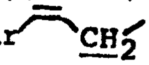$CH_2$ +Ar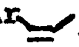$CH_2$-$C_5H_{11}$), 3.3(1H, t, J=7.4Hz, $CH(CO_2Et)_2$), 4.7(4H, m, $(CO_2CH_2CH_3)_2$), 5.66(2H, m, Ar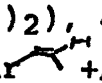 +Ar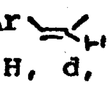 ), 6.36(1H, d, J=11.5Hz, 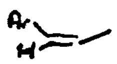 $C_6H_{13}$), 6.46(1H, d, J=11.5Hz, Ar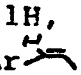 ), 7.2(4H, m, aromatic); M$^{+\cdot}$ 400.2615, $C_{25}H_{36}O_4$ requires 400.2613.

Found C, 74.76; H, 9.14; $C_{25}H_{36}O_4$ requires; C, 74,96; H, 9.06%.

0207599

## Example 15

## 2-(4-[2-(Z)-1-Octenyl)phenyl-(Z)-but-3-enyl)malonic acid

Diethyl-2-(4-[2-((Z)-1-octenyl)phenyl]-(Z)-but-3-enyl) malonate (0.6g, 1.5mmole) from Example 14 was treated with lithium hydroxide monohydrate (0.16g, 2.2eq) according to the procedure of Example 13. Chromatography on reverse phase silica (90% MeOH/$H_2O$) yielded the title compound (0.41g, 80%) as an oil. νmax(film) 3300(s,br), 2950(s), 1720(s), 910(m) and 730(m)$cm^{-1}$;δ ($CDCl_3$) 0.85(3H, t, J=7.1Hz, terminal $CH_3$), 1.25(8H, m, alkylene chain), 2.01(2H, q, J=7.7Hz, $\underline{CH_2}CH(CO_2H)_2$), 2.14(2H, q, J=7.42Hz,=-$\underline{CH_2}$-$C_5H_{11}$), 2.23 (2H, q, J=7.15Hz,=-$\underline{CH_2}$-$C_4H_5O_4$), 3.38(1H, t, J=7.4Hz,-$\underline{CH}(CO_2H)_2$), 5.65(2H, m, Ar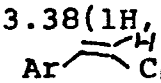$C_5H_7O_4$, Ar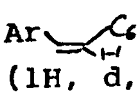), 6.35(1H, d, J=11.5Hz, 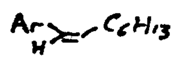 ), 6.50 (1H, d, J=11.5Hz, Ar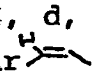$C_5H_7O_4$), 7.18(4H, m, aromatic), 8.5(2H, br, $CO_2\underline{H}$);

Found C, 69.64; H, 8.16; $C_{21}H_{28}O_4 \cdot H_2O$ requires C, 69.59; H, 8.34%.

## Example 16

## 2-(1-octynyl)bromobenzene

Dibromobenzene (11.8g, 50mmole) from Example 14 was reacted with 1-octyne (10g, 1.8eq) in the presence of bis(triphenylphosphine)palladiumdichloride (1.25g) and copper (I) iodide (0.72g) according to the procedure of Example 1. Chromatography on silica (Hexane) yielded the title compound (9.72g, 73%).$\nu$ max(film)2930(s), 2230(w), 1590(w), 1465(m), 1030(m), 750(s);$\delta$ ($CDCL_3$) 0.73(3H, t, J=6.5Hz, terminal ($CH_3$), 1.1-1.8(8H, m, alkylene chain), 1.43(2H, t, J=6Hz, $\equiv$-$\underline{C}H_2$-$C_5H_{11}$), 6.9-7.7(4H, m, aromatic); $M^{+\cdot}$ 264.0516, $C_{14}H_{17}Br$ requires 264.0514. The reaction also yielded di (1-octynyl)benzene(1.8g, 12.2%). $\nu$ max (film) 2880(s), 2220(w), 1600(w) and 755(m)$cm^{-1}$;$\delta$ ($CDCl_3$) 0.93(6H, t, J=6.5Hz, terminal $CH_3$), 1.1-1.9(16H, m, alkylene chain), 2.45(4H, t, J=6.5Hz, $\equiv$-$C\underline{H}_2$-$C_5H_{11}$), 7.05-7.5(4H, m, aromatic).

Example 17

2-(1-octynyl)bromobenzene

To 1-octyne (1.1g, 10mmole) in THF (10ml) at -78$^{o}$C was added n-butyl lithium (1.05eq). The mixture was warmed to 0$^{o}$C over ½hr were upon a solution of zinc chloride (1.3g, 10mmole) in THF (10ml) was added and stirring was continued for ½hr during which time the temperature was raised to room temperature. Sequentially -dibromobenzene (2.36g, 10mmole) in THF (5ml) and tetrakis(triphenylphosphine)palladium(0) (0.58g, 5%) in THF (10ml) were added. The mixture was heated to 60$^{o}$C overnight after which the mixture was cooled and added to dilute HCl. The product was extracted into diethyl ether and the ethereral solution was dried, ($MgSO_4$), and concentrated under reduced pressure. Chromatography on silica (Hexane) yielded the title compound (2.13g, 80.4%) identical to that obtained in Example 16.

**Example 18**

**4-[2-(1-Octynyl)phenyl]-3-butyn-1-ol tert butyl dimethylsilyl ether**

2-(1-Octynyl)bromobenzene (5.3g, 20mmole) from Example 16 was reacted with butyn-1-ol tert butyldimethylsilyl ether (5.53g, 30mmole) in an identical manner to Example 17. Chromatography on silica (Hexane) yielded the title compound (4.58g, 70%). $\nu$ max(film) 2920(s), 2850(s), 2270(w), 1595(w), 1100(s) and 835(s)$cm^{-1}$; $\delta$ ($CDCl_3$) 0.00(6H, s, $(CH_3)_2Si$), 0.83(12H, m, $(\underline{CH_3})_3CSi$; + terminal $CH_3$), 1.20-1.80(8H, m, alkylene chain), 2.37(2H, t, J=6.6Hz), $\equiv$-$\underline{CH_2}$-$C_5H_{11}$), 2.57(2H, t, J=7.5Hz, $\equiv$-$\underline{CH_2}CH_2OR$), 3.77(2H, t, J=7.5Hz, $\underline{CH_2}OR$), 7.25(4H, m, aromatic); $M^{\dagger}$ 353.2306, $C_{23}H_{33}OSi$ requires 353.2301.

Found C, 78.22; H, 9.72; $C_{23}H_{33}OSi$ requires C, 78.19; H, 9.85%.

Example 19

4-[2-(1-Octynyl)phenyl]-3-butyn-1-ol

Tetra n-butylammoniumflouride (1.5eq, 6.15g) was added to 4-[2-(1-octynyl]-3-butyn-1-ol tert-butyldimethylsilyl ether (4.24g, 13mmole), from Example 18, in THF (50ml) at 0°C under nitrogen. After warming to room temperature and stirring for 1hr, the solvent was removed under reduced pressure and the resulting oil chromatographed on silica (DCM) to yield the title compound (2.27g, 69%). ν max(film) 3340(s, br), 2925(s), 2850(m), 2220(w), 1595(w), 1480(s), 1250(m), 1040(s), 815(s) and 750(s)$cm^{-1}$; δ($CDCl_3$) 0.9(3H, t, J=6Hz, terminal $CH_3$), 1.1-1.8(8H, m, alkylene chain), 2.2(1H, s, OH), 2.4(2H, t, J=6Hz, ≡-$CH_2$-$C_5H_{11}$), 2.7(2H, t, J=7Hz, ≡-$CH_2CH_2OH$), 3.7(2H, t, J=7Hz, $CH_2OH$), 7.0-7.6(4H, m, aromatic);

Found C, 85.04; H, 8.84; $C_{18}H_{22}O$ requires C, 84.99; H, 8.72%.

## Example 20

## Tert butylethyl-2-(4-[2-(1-octynyl)phenyl]-but-3-ynyl) malonate

4-[2-(1-Octynyl)phenyl]-3-butyn-1-ol p-toluenesulphate (2.04g, 5mmole), from Example 11, was treated with tert butylethylmalonate (2.36g, 10mmole) according to the procedure of Example 12. Chromatography on silica (Hexane: DCM, 1:1) yielded the title compound (0.52g, 26%) as an oil. ν max(film) 2925(s), 2220(w), 1750(s), 1730(s), 1595(w) and 750(m)$cm^{-1}$; δ($CDCl_3$) 0.90(3H, t, J=6.6Hz, terminal $CH_3$), 1.25-1.35(9H, m, alkylene chain + ethylester Me), 1.47 (9H, s, $(CH_3)_3C$), 1.61(2H, m, ≡-$CH_2$<u>$CH_2$</u>-$C_4H_9$), 2.18(2H, q, J=7.25Hz, <u>$CH_2$</u>$CH(CO_2Et)(CO_2tBu)$), 3.60(1H, t, J=7.7Hz, <u>CH</u>$(CO_2Et)(CO_2tBu)$), 4.20(2H, m, ethylester $CH_2$), 7.18(2H, m, aromatic), 7.37(2H, m, aromatic); $M^{+\cdot}$ 424.2623, $C_{27}H_{36}O_4$ requires 424.2613.

Found C, 76.18; H, 8.55; $C_{27}H_{36}O_4$ requires C, 76.38; H, 8.55%.

Also formed in the reaction was 1-[2-(1-octynyl)phenyl) -but-3-yn-1-ene(0.60g, 51%) identical to that obtained in Example 12.

## Example 21

## 2-(4-[2-(1-Octynyl)phenyl]-but-3-ynyl)malonic acid monotertbutyl ester

Tertbutylethyl-2-(4-[2-(1-octynyl)phenyl]-but-3-ynyl malonate (0.50g, 1.18mmole) from Example 20 was treated with lithium hydroxide monohydrate (0.05g, 1eq) according to the procedure of Example 13. Chromatography on silica (Hexane:diethylether) yielded the title compound (0.29g, 62%) as an oil. $\nu$ max (film) 3100 (m, br), 2220(w), 1735(s), 1710(s), 1590(w), 1150(s) and 755(m)$cm^{-1}$; $\delta$ ($CDCl_3$) 0.90(3H, t, J=6.87Hz, terminal $CH_3$), 1.20-1.36(6H, m, alkylene chain), 1.48(9H, s, $(\underline{CH_3})_3C$), 1.61(2H, m, $\equiv$-$CH_2\underline{CH_2}C_4H_9$), 2.20(2H, q, J=7.01Hz, $\underline{CH_2}CH(CO_2H)(CO_2tBu)$), 2.47(2H, t, J=7.15Hz, $\equiv$-$CH_2$-$C_5H_{11}$), 2.59(2H, t, J=6.87Hz, $\equiv$-$\underline{CH_2}$-$C_8H_{13}O_4$), 3.67(1H, t, J=7.43Hz, $\underline{CH}(CO_2H)(CO_2tBu)$), 7.18(2H, m, aromatic), 7.38(2H, m, aromatic); $M^{+}$ 396.2307, $C_{25}H_{32}O_4$ requires 396.2300.

Found C,75.89; H, 8.33; $C_{25}H_{32}O_4$ requires C,75.72; H, 8.14%.

Also isolated by chromatography was tert-butylethyl-2-(4-[2-(1-octynyl)phenyl]-but-3-ynyl)malonate(0.12g, 24%) identical to the material obtained in Example 20.

**Example 22**

**Methyl-6-[2-(1-octynyl)phenyl]hex-5-yn-1-oate**

2-(1-Octynyl)bromobenzene (5.3g, 20mmole) from Example 16 was reacted with methylhex-5-yn-1-oate (3.16g, 25mmole) in an identical manner to Example 17. Chromatography on silica (DCM: Hexane 1:1) yielded the title compound (2.02g, 26.5%). ν max(film) 2975(s), 2220(w), 1740(s), 1595(w) and 760(m)$cm^{-1}$; δ ($CDCl_3$) 0.90(3H, t, J=6.8Hz, terminal $CH_3$), 1.25-1.68(8H, t, alkylene chain), 1.95(2H, pent, J=7.15, $\equiv$-$CH_2\underline{CH_2}CH_2CO_2Me$), 2.46(2H, t, J=7.15,$\equiv$-$\underline{CH_2}$-$C_5H_{11}$), 2.55(2H, t, J=6.87Hz,$\equiv$-$\underline{CH_2}CH_2CH_2CO_2Me$), 2.58(2H, t, J=7.43Hz, $\underline{CH_2}CO_2Me$), 3.68(3H, s, ester Me), 7.18(2H, m, aromatic), 7.36(2H, m, aromatic).

Found C, 81.63; H, 8.42; $C_{21}H_{26}O_2$ requires C, 81.25; H, 8.44%.

Also isolated by chromatography was 2-(1-octynyl) bromobenzene(1.22g, 23%). Identical with material obtained in Example 16.

Example 23

6-[2-(1-Octynyl)phenyl)hex-5-yn-1-oic acid

Methyl-6-[2-(1-octynyl)phenyl]hex-5-yn-1-oate(0.78g, 2.5mmole) from Example 22 was stirred with lithium hydroxide monohydrate (0.16g, 1.5eq) according to the method of Example 13. Chromatography on reverse phase silica (95% MeOH/$H_2O$) yielded the title compound (0.70g, 95%) as an oil. $\nu$ max(film) 3000(m, br), 2220(w), 1710(s), 1595(w) and 750(m); $\delta$ ($CDCl_3$) 0.90(3H, t, J=6.6Hz, terminal $CH_3$), 1.29-1.53(6H, m, alkylene chain), 1.62(2H, m, $\equiv$-$CH_2\underline{CH_2}C_4H_9$), 1.95(2H, m, $\underline{CH_2}CO_2H$), 2.46(2H, t, J=7.15, $\equiv$-$CH_2$-$C_5H_{11}$), 2.57(2H, t, J=6.88Hz, $\equiv$-$CH_2CH_2CH_2CO_2H$), 2.64(2H, t, J=7.43, $\underline{CH_2}CO_2H$), 7.18(2H, m, aromatic), 7.36(2H, m, aromatic); $M^{+\cdot}$ 296.1775, $C_{20}H_{24}O_4$ requires 296.1776.

Found C, 81.20; H, 8.06; $C_{20}H_{24}O_4$ requires C, 81.04; H, 8.16%.

## Example 24

## Methyl-6-[2-((Z)-1-octenyl)phenyl]-(Z)-hex-5-en-1-oate

Methyl-6-[2-(1-octynyl)phenyl]hex-5-yn-1-oate(1.09g, 3.5mmole) from example 22 was hydrogenated in the presence of 5% palladium on barium sulphate (0.15g) according to the method of Example 3. Chromotography on silica (hexane: DCM, 2:1) yielded the title compound (0.85g, 77.3%) as an oil. $\nu$ max(film) 2925(s), 1740(s), 1640(w), 1595(w) and 765(m)$cm^{-1}$; $\delta$ ($CDCl_3$) 0.86(3H, t, J=7.15Hz, terminal $CH_3$), 1.25-1.64(10H, m, alkylene chain + $\underline{CH_2}CH_2CO_2Me$), 1.73(2H, m, =-$\underline{CH_2}C_5H_{11}$), 2.10-2.23(4H, m=-$\underline{CH_2}CH_2\underline{CH_2}CO_2Me$), 5.64(1H, dt, Jd=11.54Hz, Jt=7.42Hz, 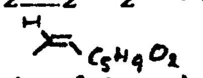 ), 5.68(1H, dt, Jd=11.54Hz, Jt=7.42Hz, 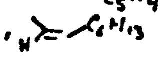 ), 6.36(1H, d, J=11.27Hz, 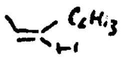 ), 6.44(1H, d, J=11.55Hz, 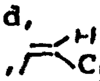$C_5H_9O_2$), 7.11-7.26(4H, m, aromatic); $M^{+\cdot}$ 314.2250, $C_{21}H_{30}O_2$ requires 314.2246.

Found C, 80.20; H, 9.82; $C_{21}H_{30}O_2$ requires C, 80.21; H, 9.62%.

**Example 25**

**6-[2-((Z)-1-octenyl)phenyl]-(Z)-hex-5-en-1-oic acid**

Methyl-6-[2-((Z)-1-octenyl)phenyl]-(Z)-hex-5-en-1-oate (0.785g, 2.5mmole) from Example 24 was treated with lithium hydroxide monohydrate (0.22g, 2eq) according to the procedure of Example 13. Chromatography on reverse silica (90% MeOH/$H_2O$) yielded the title compound (0.7g, 93%) as an oil. ν max(film) 3000 (m, br), 1705(s), 1595(w) and 760cm$^{-1}$; δ $CDCl_3$) 0.85(3H, t, J=7.15, terminal $CH_3$), 1.23-1.4(10H, m, alkylene chain), 1.73(2H, pent, J=7.56Hz, $\underline{CH_2}CH_2CO_2H$), 2.08-2.25(4H, m, $=-\underline{CH_2}C_5H_{11}+ =-\underline{CH_2}-C_3H_5O_2$), 2.32(2H, t, J=7.7Hz, $\underline{CH_2}CO_2H$), 5.66(2H, m, 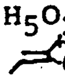$C_6H_{13}$ + 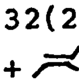$C_4H_7O_2$), 6.36(1H, d, J=11.55, 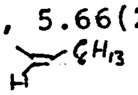), 6.45(1H, d, J=11.54, 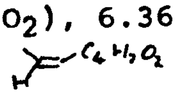), 7.11-7.24(4H, m, aromatic); M$^{+}$ 300.2092, $C_{20}H_{28}O_2$ requires 300.2089.

Found C, 80.10; H, 9.50; $C_{20}H_{28}O_2$ requires C, 79.96; H, 9.39%.

**Example 26**

**6-[2-(1-Octynyl)phenyl]hex-5-yn-1-oic acid**

Diethyl-2-(4-[2-(1-octynyl)phenyl]-but-3-ynyl)malonate (0.43g, 1.245mmole) from Example 12 was heated to 100°C when gas evolution was observed. When gas evolution ceased the mixture was cooled and was chromatographed on reverse phase silica (diethyl ether:hexane) to yield the title compound (0.070g, 19%) identical to material obtained in Example 23 and diethyl-2-(4-[2-(1-octynyl) phenyl]-but-3-ynyl)malonate (0.13g, 30%) identical with material obtained in Example 12.

Example 27

6-[2-(1-Octynyl)phenyl]-5-hexyn-1-ol tertbutyl dimethylsilyl ether

n Butyl lithium (5.78mmole) in hexane was added to hex-5-yn-1-oltert-butyldimethylsilyl ether(1.17g, 5.5mmole) in THF (4ml) at -30°C. The solution was stirred for ½hr and allowed to warm to room temperature when a solution of zinc chloride (0.75g) in THF (5ml) was added. After a further ½hr a solution of 2-(1-octynyl) bromobenzene from Example 16 (1.22g, 4.6mmole) in THF (2ml) and a solution of tetrakis (triphenylphosphine)palladium(0)(0.26g, 5%) in THF (5ml) were added sequentially. The mixture was heated to reflux for 36hr after which it was added to dilute hydrochoric acid and the acid was extracted into diethyl ether. After drying, the ethereal solution was evaporated and the resulting oil was chromatographed on silica (DCM:hexane 1:1) to give 2-(1-octynyl) bromobenzene (0.3g, 24.6% identical to material obtained in Example 16 and the title compound (0.45g, 25.6%). $\nu$max(film) 2950(s), 2215(w), 1590(w),1460(m), 1250(m), 1110(s) and 835(s)$cm^{-1}$; $\delta$($CDCl_3$) 0.00(6H, s, $(\underline{CH_3})_2Si$), 0.84(9H, s, $(CH_3)_3CSi$), 0.85(3H, t, J=6.7Hz, terminal $CH_3$), 1.20-1.69(12H, m, alkylene chain + $\underline{CH_2}\underline{CH_2}CH_2O$-), 2.40(2H, t, J=6.96Hz, $\equiv$-$CH_2$-$C_5H_{11}$), 2.43(2H, t, J=6.68Hz, $\equiv$-$\underline{CH_2}C_9H_{21}OSi$), 3.61(2H, t, J=6.00Hz, $\underline{CH_2}OSiC_6H_{15}$), 7.10-7.15(2H, m, aromatic), 7.20-7.33(2H, m, aromatic); $M^{+\cdot}$ 396.2838, $C_{26}H_{40}OS$; requires 396.2848.

0207599

**Example 28**

**6-[2-(1-Octynyl)phenyl]-5-hexyn-1-ol**

To 6-[2-(1-octynyl)phenyl]-5-hexyn-1-ol tertbutyl dimethylsilyl ether (0.396g, 1mmole) from Example 27 in THF (5ml) at 0°C under nitrogen was added tetra n-butyl ammoniumfluoridetrihydrate (0.47g, 1.5eq). The temperature was allowed to warm to room temperature and stirring was continued for 4hr. The solvent was removed under reduced pressure and the residue was chromatographed on silica (DCM) to give the title compound (0.26g, 92%).ν max(film) 3340(s, br), 2930(s), 2225(w), 1595(w), 1480(m), 1065(s) and 760(s)$cm^{-1}$; δ($CDCl_3$) 0.90(3H, t, J=6.78Hz), 1.29-1.84(13H, m, alkylene chain + $\underline{CH_2}\underline{CH_2}CH_2\underline{OH}$), 2.46(2H, t, J=6.96Hz, $\equiv$-$\underline{CH_2}$-$C_5H_{11}$), 2.51(2H, t, J=6.78Hz, $\equiv$-$\underline{CH_2}$ $C_3H_7O$), 3.71(2H, t, J=6.14Hz, $\underline{CH_2}OH$), 7.14-7.19(2H, m, aromatic), 7.21-7.40(2H, m, aromatic); M. 282.1980, $C_{20}H_{26}O$ requires 282.1983.

Found C, 85.13; H, 9.09; $C_{20}H_{26}O$ requires C, 85.05; H, 9.28%.

## Method For The Measurement of Inhibition of Phospholipase $A_2$ Activity

Enzyme: Phospholipase $A_2$ (Naja naja venom , Sigma)

Substrate: Diarachidoyl phosphatidylcholine (10.6mg) and α-1-palmitoyl-2-[1-$^{14}$C]-arachidonyl phosphatidylcholine (0.5μCi; 52.9mCi/mMol) in 28ml of Tris HCl buffer = 100mM, pH 8.7) ultrasonicated.

Assay: Incubations containing $PLA_2$ (0.125ng) in Tris HCl buffer (100mM, pH 8.7) and $CaCl_2$ (4mM) were prepared on ice. Immediately prior to incubation at 37$^{o}$C 5μl of DMSO or compound in DMSO were added. The final assay volume was 1.75ml. After 10 minutes incubation at 37$^{o}$C 750μl of substrate was added, with mixing, and the incubation continued for a further 15 min. The reaction is terminated by placing on ice and adding 5ml $CHCl_3$:MeOH (2:1v/v).

The lower organic phase is removed and stored under $N_2$ at -20$^{o}$C until analysed. Analysis is by 1-dimensional t.l.c. on polygram Sil G sheets in the solvent system: petroleum ether: diethyl ether: acetic acid (60:40:1, v/v). In this solvent system the phospholipid remains at the origin and the arachidonic acid has an approximate Rf. value of 0.36. The areas of interest are cut from the plates and their radioactivity determined by liquid scintillation counting.

All compounds are tested in triplicate.

Calculation: The radioactivity of the arachidonic acid is expressed as a percentage of the total dpm cut from the plate. The percentage inhibition of arachidonic acid release compared to that of the control is calculated after correction for

the level of spontaneous release where:

$\bar{x}$ = % corrected mean arachidonic acid release in control

y = % corrected arachidonic acid release after inhibition

$$\% \text{ inhibition } (z) = \frac{\bar{x} - y}{\bar{x}} \times 100$$

The mean of the triplicate samples is calculated giving $\bar{z}$ ± standard deviation %.

Biological Results

| Example No. | Percent inhibition of phospholipase $A_2$ at x μM concentration. |
|---|---|
| 2 | 76.2± 13.9 at 20μM |
| 3 | 72.9± 10.3 at 20μM |
| 5 | 78.9± 4.7 at 20μM |
| 13 | 23.1± 17.6 at 20μM |
| 15 | 65.0± 17.0 at 20μM |
| 21 | 62.0± 4.4 at 20μM |
| 23 | 25.0± 19.0 at 20μM |
| 25 | 69.8± 8.3 at 20μM |

## CLAIMS

1. A compound of formula (I):

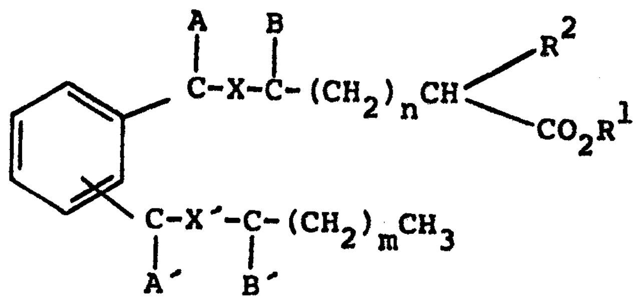

(I)

or where appropriate a salt, amide or solvate thereof,

wherein $R^1$ is hydrogen or $C_{1-6}$ alkyl;
$R^2$ is hydrogen or $CO_2R^3$ where $R^3$ is hydrogen or $C_{1-6}$ alkyl;
X and X′ are independently a double or triple bond;
A and B are hydrogen when X is a double bond or A and B are both absent when X is a triple bond;
A′ and B′ are hydrogen when X′ is a double bond or A′ and B′ are both absent when X′ is a triple bond;
n is 0 or an integer of from 1 to 4; and m is an integer of from 2 to 7.

2. A compound as claimed in claim 1, characterized in that $R^1$ is hydrogen or ethyl.

3. A compound as claimed in claim 1 or 2, characterized in that $R^2$ is hydrogen, $-CO_2H$, $-CO_2CH_3$ $-CO_2CH_2CH_3$ or $-CO_2Bu$.

4. A compound as claimed in claim 1, 2 or 3, characterized in that n is 2.

5. A compound as claimed in any one of claims 1 to 4, characterized in that m is 4 or 5.

6. A compound as claimed in claim 1, selected from the list consisting of:
6-[2-(1-octynyl)phenyl]-E-hex-5-en-1-oic acid;
6-[2-(Z-1-octenyl)phenyl]-E-hex-5-en-1-oic acid;
6-[3-(1-octynyl)phenyl]-E-hex-5-en-1-oic acid;
2-(4-[2-(1-octynyl)phenyl]-but-3-ynyl)malonic acid;
2-(4-[2-(Z)-1-octenyl)phenyl-(Z)-but-3-enyl)-malonic acid;
2-(4-[2-(1-octynyl)phenyl]-but-3-ynyl)malonic acid monoterbutyl ester;
6-[2-(1-octynyl)phenyl)hex-5-yn-1-oic acid;
6-[2-((Z)-1-octenyl)phenyl]-(Z)-hex-5-en-1-oic acid;
diethyl 2-(4-[2-(1-octynyl)phenyl]- but-3-ynyl)-malonate;
diethyl-2-(4-[2-((Z)-1-octenyl)phenyl]-(Z)-but-3-enyl)malonate;
tert butylethyl-2-(4-[2-(1-octynyl)phenyl]-but-3-ynyl)malonate; and
methyl-6-[2-((Z)-1-octenyl)phenyl]-(Z)-hex-5-en-1-oate;
or, where appropriate, a salt, amide or solvate thereof

7. A process for preparing a compound as claimed in claim 1 which comprises:

A) for compounds of formula (I) wherein X is a double bond and A and B are hydrogen, reacting a compound of formula (II):

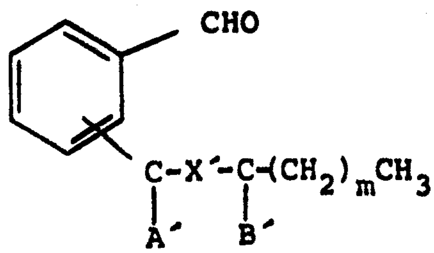

(II)

wherein X′, A′, B′ and m are as defined in relation to formula (I) with a compound of formula (III):

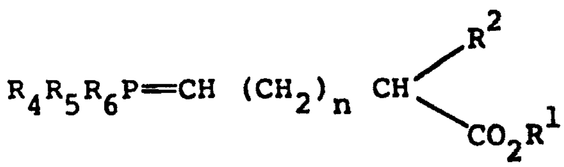

(III)

wherein $R^1$ is hydrogen and $R^2$ and n are as defined in relation to formula (I), and $R^4$, $R^5$ and $R^6$ are each independently $C_{1-6}$ alkyl or aryl; in the presence of a strong base,

B) deprotecting a compound of formula (VII):

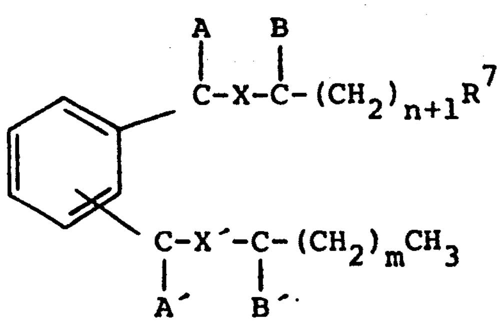

(VII)

wherein X, A, B, X′, A′, B′, m and n are as defined for formula (I) and $R^7$ is a protected acid group;

C) reacting a compound of formula (XIX):

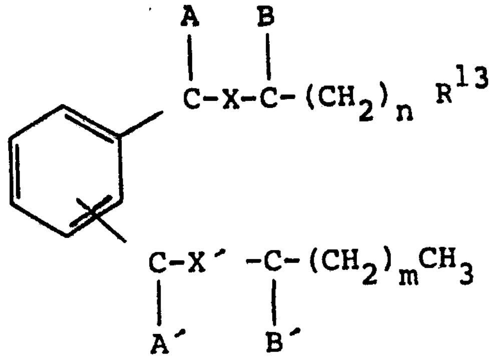

(XIX)

wherein A,B,X,A′,B′,X′, n and m are as defined in relation to formula (I) and $R^{13}$ is a leaving group with a source of malonate ions of formula (XX):

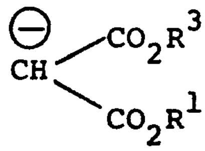

(XX)

D) oxidation of a compound of formula (XXII):

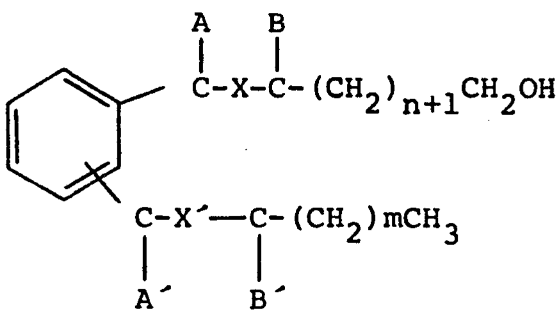

(XXII)

wherein A,B,X,A′,B′,X′ n and m are as defined in relation to formula (I); or

E) reaction of a compound of formula (XXIII):

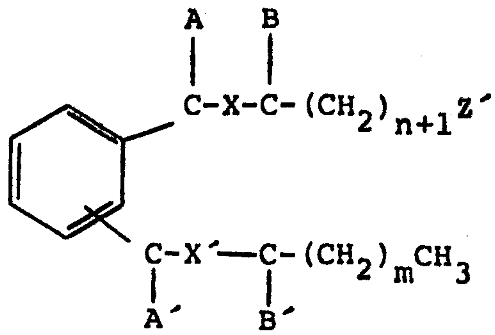

(XXIII)

where A,B,X,A′,B′,X′, m and n are as defined in relation to formula (I) and Z' is a metal or metal salt ion with carbon dioxide, or a suitable chloroformate; and thereafter if necessary carrying out one or more of the following steps:

(i) where X and/or X' is a triple bond, reducing the triple bond to a double bond;

(ii) converting one group $R^1$ to another group $R^1$;

(iii) converting one group $R^2$ to another group $R^2$; and

(iv) forming a salt, amide or solvate thereof.

8. A pharmaceutical composition comprising a compound of formula (I), as claimed in claim 1, or a pharmaceutically acceptable salt, amide or solvate thereof, and a pharmaceutically acceptable carrier therefor.

9. A compound of formula (I), or a pharmaceutically acceptable salt, amide or solvate thereof, for use as an active therapeutic substance.

10. The use of a compound of formula (I), as defined in claim 1, or a pharmaceutically acceptable salt, amide or solvate thereof for the manufacture of a medicament for the treatment of allergic and/or inflammatory diseases.

European Patent Office

# EUROPEAN SEARCH REPORT

0207599
Application number

EP 86303403.9

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,A | EP - A1 - 0 109 225 (BEECHAM)<br>* Claims *<br>-- | 1,7-10 | C 07 C 57/42<br>C 07 C 69/618<br>C 07 C 51/00 |
| P,A | EP - A2 - 0 142 145 (BEECHAM)<br>* Claims *<br>---- | 1,7-10 | C 07 C 67/00<br>A 61 K 31/19<br>A 61 K 31/215 |
| | | | TECHNICAL FIELDS SEARCHED (Int. Cl.4) |
| | | | C 07 C 57/00<br>C 07 C 69/00 |

The present search report has been drawn up for all claims

| Place of search | Date of completion of the search | Examiner |
|---|---|---|
| VIENNA | 04-08-1986 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82